# EUROPEAN PATENT APPLICATION

(11) **EP 2 886 663 A1**
(43) Date of publication of application: **24.06.2015**
(21) Application number: 13306795.9
(22) Date of filing: 19.12.2013
(51) Int. Cl.: C12Q 1/68

(54) **Nanopore sequencing using replicative polymerases and helicases**

(71) Applicant: Centre National de la Recherche Scientifique (CNRS), 75016 Paris (FR); Ecole Normale Supérieure, 75005 Paris (FR); Universite d'Evry Val d'Essonne, 91000 Evry (FR)
(72) Inventor: Croquette, Vincent, 92160 Antony (FR); Manosas, Maria, 08037 Barcelona (ES); Mathe, Jerome, 91240 Saint Michel S/Orge (FR); Bensimon, David, 75013 Paris (FR); Allemand, Jean-Francois, 92340 Bourg La Reine (FR)
(74) Representative: Regimbeau

(57) **Abstract**

The present invention relates to a system of nanopore sequencing using nucleic-acid binding proteins wherein the activity of the said protein and the passage of the sequenced nucleic acid molecule through the nanopore are coordinated. In particular, the present invention enables an easy synchronization of nanopore capture with polynucleotide unwinding, which in turn affords an easy way to repeat the whole process of capture and nucleotide characterization.

## Description

### Introduction

The present invention relates to a fast method for the determination of a sequence of a nucleic acid, DNA or RNA, which is useful, in particular, for the sequencing of an unknown nucleic acid or alternatively for the detection of a specific nucleic acid sequence for diagnosis.

The high demand for low-cost sequencing has driven the development of high-throughput sequencing technologies that parallelize the sequencing process, producing thousands or millions of sequences at once (Shendure 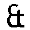 Ji, Nat Biotechnol., 26(10): 1135-45. 2008). High-throughput sequencing technologies are intended to lower the cost of DNA sequencing beyond what is possible with standard dye-terminator methods. Such methods are described in e.g. US Patent No 4,882,127, U.S. Patent No. 4,849,077; U.S. Patent No.7,556,922; U.S. Patent No. 6,723,513; PCT Patent Application No. WO 03/066896; PCT Patent Application No. WO2007111924; U.S. Patent Application No. US 2008/0020392; PCT Patent Application No. WO 2006/084132; U.S. Patent Application No. US 2009/0186349; U.S. Patent Application No. US 2009/0181860; U.S. Patent Application No. US 2009/0181385; U.S. Patent Application No. US 2006/0275782; European Patent EP-B1-1141399; Shendure 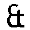 Ji, Nat Biotechnol., 26(10): 1135-45. 2008; Pihlak et al., Nat Biotechnol., 26(6) : 676-684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

However, all the methods developed so far suffer from serious drawbacks. A new single-strand sequencing method based on the use of nanopores, i.e. nanoscale pores, has been recently developed and deals with most shortcomings of current sequencing platforms: reads are very long; errors are random rather than bunched together at the end of a read; data can be read in real time; throughput is high; and input DNA is not destroyed in the process (Branton et al., Nature Biotechnol., 26(10): 1146-1153, 2008).

According to this method, a nanopore spanning across an impermeable thin membrane is placed between two chambers that contain an electrolyte, and voltage is applied across the membrane using two electrodes. These conditions lead to a steady stream of ion flow across the pore. A single stranded DNA (ssDNA) molecule can be driven by electrophoresis through the nanopore; as the molecule translocates through the hole, the ionic current flowing in the hole is reduced depending upon the nature of the base in the pore. Reading the current versus time provide a way to deduce the sequence (Kasianowicz, Proc Natl Acad Sci USA, 93: 13770-13773, 1996).

The method suffers severe limitations, since uncontrolled DNA strand electrophoresis through nanopores is too fast for accurate base reads (Branton et al., Nature Biotechnol., 26(10): 1146-1153, 2008; Wanunu, Phys Life Rev., 9(2): 125-158, 2012).

Substantial reductions of the translocation rate can be achieved with processive DNA enzymes, which limit the translocation rate by binding to the DNA strand and preventing it from moving into the narrow confined of the pore faster than the enzyme processing rate (see e.g. Benner et al., Nat Nanotechnol., 2(11): 718-724, 2007; Olasagasti et al., Nat Nanotechnol., 5(11): 798-806, 2010; Lieberman et al., J. Am. Chem. Soc., 132: 17961-17972, 2010; Venkatesan 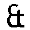 Bashir, Nat Nanotechnol., 6(10): 615-624, 2011; Cherf et al., Nat. Biotechnol., 30(4): 344-348, 2012; Manrao et al., Nat Biotechnol., 30(4):349-353, 2012). However, under normal conditions, such enzymes start working whenever they find their DNA substrate and do not wait for some signal.

Ingenious arrangements have been described in literature to synchronize the start of the molecular motor with the entry of the ssDNA in the nanopore. For example, variations in the level of the ATP or Mg²⁺ cofactor have been used to ensure that the working complex is assembled just at the nanopore (WO 2008/124107).

Alternative methods rely on the phi29 DNA polymerase. This enzyme is particularly suitable to this approach because it remains bound to DNA, even against the force of an applied voltage needed to insert the DNA into the pore. For example, a first study used transient chemical protection of the DNA primer to prevent elongation and excision in bulk phase (Lieberman et al., J. Am. Chem. Soc., 132: 17961-17972, 2010). Another study was based on using a blocking oligonucleotide which hybridized downstream of primer and which was displaced once the single-stranded DNA was captured by the pore (Cherf et al., Nat. Biotechnol., 30(4): 344-348, 2012; Manrao et al., Nat Biotechnol., 30(4):349-353, 2012).

However none of these approaches is fully satisfactory. All of them are technically challenging, and therefore cannot be easily applied to high throughput sequencing. In addition, these approaches are dependent upon polymerization of a new nucleic acid strand, which leads to insertion errors and therefore poor accuracy. Indeed, the error rate is about 4%, whereas the current high throughput sequencing methods are 99.99% accurate (Cherf et al., Nat. Biotechnol., 30(4): 344-348, 2012; Schneider 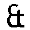 Dekker, Nat. Biotechnol., 30(4): 326-328, 2012; Liu et al., J Biomed Biotechnol, 2012: 251364, doi: 10.1155/2012/251364, 2012). Moreover it is not possible to make the same DNA molecule cross the pore several times and thus repeat the measure to improve sensitivity.

There is thus a need for a for single-molecule nucleic-acid sequencing method which is both reliable and accurate.

### Description

The present invention relates to a system of nanopore sequencing using nucleic-acid binding proteins wherein the activity of the said protein and the passage of the sequenced nucleic acid molecule through the nanopore are coordinated. In particular, the present invention enables an easy synchronization of nanopore capture with polynucleotide unwinding, which in turn affords an easy way to repeat the whole process of capture and nucleotide characterization.

The present inventors have surprisingly found that the enzymatic activity of some nucleic-acid binding proteins can be activated by application of an external force to a nucleic acid molecule. When no force is applied, the said proteins remain inactive. Thus, it is possible to control the activity of a nucleic-acid binding protein loaded onto a polynucleotide by capturing one end of the said polynucleotide by a nanopore, thus applying a tension to the said polynucleotide. When said tension is applied, the said protein is activated, leading to the movement of a strand of the polynucleotide with respect to the nanopore. This movement generates a signal which is dependent upon the nature of the nucleotides.

In a first embodiment, the invention relates to a method of sequencing a polynucleotide, said method comprising the steps of:
a) contacting said polynucleotide with a nanopore and a nucleic-acid binding enzyme;
b) introducing said polynucleotide into the said nanopore, resulting in the activation of the activity of the said enzyme;
c) allowing said polynucleotide to move with respect to the said nanopore, wherein the movement of said polynucleotide is controlled by the activity of the said enzyme;
d) monitoring the signal associated with the movement of the said polynucleotide with respect to the said nanopore, thereby generating the sequence of the said polynucleotide;
e) switching off the activity of the said enzyme; and
f) repeating steps a) to e).

A "polynucleotide" according to the invention is a macromolecule comprising two or more nucleotides. The polynucleotide or nucleic acid (these terms are used synonymously throughout the present specification and should be construed as carrying the same meaning) of the invention comprises any combination of nucleotides. A polynucleotide as used herein refers to DNA or RNA, including any naturally occurring, synthetic, or modified nucleotide.

As generally used herein, a "nucleotide" or "base" can be a naturally-occurring nucleotide or a nucleotide analog. A naturally-occurring nucleotide is deoxyadenosine mono-phosphate (dAMP), deoxycytidine mono-phosphate (dCMP), deoxyguanosine mono-phosphate (dGMP), deoxythymidine mono-phosphate (dTMP), adenosine mono-phosphate (AMP), cytidine mono-phosphate (CMP), guanosine mono-phosphate (GMP) or uridine mono-phosphate (UMP). A nucleotide analog is an analog or mimic of a primary nucleotide having modification on the primary nucleobase (A, C, G, T and U), the deoxyribose/ribose structure, the phosphate group of the primary nucleotide, or any combination thereof. For example, a nucleotide analog can have a modified base, either naturally existing or man-made. Examples of modified bases include, without limitation, methylated nucleobases, modified purine bases (e.g. hypoxanthine, xanthine, 7-methylguanine, isodG), modified pyrimidine bases (e.g. 5,6-dihydrouracil and 5-methylcytosine, isodC), universal bases (e.g. 3-nitropyrrole and 5-nitroindole), non-binding base mimics (e.g. 4-methylbezimidazole and 2,4-difluorotoluene or benzene), and no base (abasic nucleotide where the nucleotide analog does not have a base). Examples of nucleotide analogs having modified deoxyribose (e.g. dideoxynucleosides such as dideoxyguanosine, dideoxyadenosine, dideoxythymidine, and dideoxycytidine) and/or phosphate structure (together referred to as the backbone structure) includes, without limitation, glycol nucleotides, morpholinos, and locked nucleotides.

The terms "deoxyribonucleic acid" and "DNA" as used herein mean a polymer composed of deoxyribonucleotides. The terms "ribonucleic acid" and "RNA" as used herein mean a polymer composed of ribonucleotides. The said nucleic acid may also be made of modified nucleotides, such as locked nucleic acid (LNA), which are nucleotides in which the ribose moiety is modified with an extra bridge connecting the 2' oxygen and 4' carbon, or peptide nucleic acid (PNA), wherein the backbone is composed of repeating N-(2-aminoethyl)-glycine units linked by peptide bonds.

Preferably, the polynucleotide of the invention is a double-stranded nucleic acid. In this respect, it should be emphasized that the invention applies to any type of double-stranded nucleic acid. Most often, the double-stranded nucleic acid will be DNA, but it is understood that the invention also applies to single-stranded DNA-single-stranded DNA duplexes, perfectly paired or not perfectly paired, or alternatively to single-stranded DNA-single-stranded RNA duplexes, perfectly paired or not perfectly paired, or alternatively to single-stranded RNA-single-stranded RNA duplexes, perfectly paired or not perfectly paired. Furthermore, the duplex may consist of the at least partial re-pairing of two single strands obtained from samples of different origins. Finally, the invention also applies to the secondary structures of a sole single-stranded DNA or of a sole single-stranded RNA.

When two reservoirs of solution are separated by a nanopore, which serves as a fluidic constriction of known dimensions, and a DC voltage is applied between the two reservoirs, it will result in the movement of a polynucleotide present into one reservoir through the nanopore. The capture of the said polynucleotide by the nanopore results in a force being exerted onto the said polynucleotide (Wanunu, Phys Life Rev., 9(2): 125-158, 2012). Preferably, the said exertion of force onto the polynucleotide is responsible for the activation of the nucleic-acid binding enzyme in the method of the invention.

Thus, in a preferred embodiment, step b) of the method of the invention further comprises applying a voltage to the pore. In a more preferred embodiment, the said polynucleotide in step c) moves through the said pore. In a still more preferred embodiment, the diameter of the nanopore aperture permits only a single strand of a polynucleotide to traverse the nanopore aperture at any given time. The skilled person will easily realize that, in this embodiment of the invention, the direction of the movement of the polynucleotide through the said pore can be easily determined, based on the orientation of the stand going penetrating the pore. Preferably, the direction of the movement of said polynucleotide through the said pore is 5' to 3'; alternatively, it is 3' to 5'.

In a further preferred embodiment, the movement through the pore is associated with a denaturation of the said polynucleotide. In another further preferred embodiment, the movement through the pore is associated with renaturation of the said polynucleotide. By "denaturation", it is herein meant the process of strands separation of a double-stranded nucleic acid molecule occurring when most of the hydrogen bonds between the said strands are broken. The denaturation process yields a denatured nucleic acid molecule, by which it is herein meant the two separated complementary strands resulting from the denaturation of a double-stranded nucleic acid molecule. By "renaturation", it is herein referred to the process by which two separated complementary strands reform through hybridization into a double helix. As used herein, "hybridization" is the process of establishing a non-covalent, sequence-specific interaction between two or more complementary strands of nucleic acids into a single hybrid. It will easily be understood by the skilled person that denaturation of the polynucleotide is most likely associated with a 5' to 3' movement, whereas a 3' to 5' movement most likely results in renaturation of the said polynucleotide.

Advantageously, the polynucleotide of the invention contains a fork. By "fork", it is herein referred to a region of the polynucleotide which is partly denatured. Preferably, a polynucleotide containing a fork is denatured on one side of the fork while being fully hybridized on the other side. Thus, in such a molecule, the two strands of the polynucleotide are separated on one side of the fork while being hybridized on the other side. The presence of the fork thus provides a single-stranded end of the polynucleotide which can be easily captured by the nanopore. Such a fork can be naturally present in the said polynucleotide. Alternatively, it can be artificially engineered through a linker sequence added to one extremity of the said polynucleotide. For example, two partially complementary oligonucleotides may be annealed and then linked to one end of the said polynucleotide by common molecular biology techniques.

When one free, single-stranded end of the said polynucleotide enters the nanopore after voltage is applied, a force is exerted onto the said polynucleotide which leads to the denaturation of the whole nucleic acid molecule. As a result, the fork will move along the length of the polynucleotide, thus resulting in the progressive denaturation of the said polynucleotide, from the end of the molecule which is grabbed by the pore to the other end of the molecule.

It may be advantageous to ensure that the nanopore always captures the same end of the polynucleotide. In this case, the fork will always move in the same direction, which may be required for the activity of the nucleic-acid binding enzyme. In addition, it ensures that the same strand is sequenced every time the method of the invention is repeated, thus increasing the sensitivity of the method. This can be obtained by blocking one single-stranded end of the polynucleotide.

Preferably, the polynucleotide comprises a hairpin at one end of the said polynucleotide. As used herein, 'hairpin' means a double helix wherein the 5' end of one strand is physically linked to the 3' end of another strand through an unpaired loop. The said physical link can be either covalent or non-covalent. Preferentially, the said physical link is a covalent bond. Thus, a hairpin consists of a double-stranded stem and an unpaired single-stranded loop.

The presence of a hairpin at one end of the polynucleotide is useful for preventing the capture of the said end by the nanopore. In this regard, it is particularly useful that only one extremity of the polynucleotide comprises a hairpin.

A polynucleotide containing a fork is denatured on one side of the fork while being fully hybridized on the other side and each of the two denatured strands can be captured by the pore. In order to ensure that the same strand is always captured by the pore, the extremity comprising hairpin is advantageously the one which corresponds to the denatured strands.

Preferably, the said hairpin is physically linked to the only one of the two strands of the said extremity of the polynucleotide of the invention. More preferably, the 3' end of one strand of the polynucleotide is linked to a hairpin which prevents said end from entering the pore. In this case, the only single-stranded polynucleotide end which can be captured by the nanopore is the 5' end, resulting in 5' to 3' movement of said polynucleotide through the said pore. For example, when the enzyme used in the method of the invention is a replicative polymerase, the 5' end will be captured by the pore: the hairpin should thus be linked to the 3' end. Alternatively, the 5' end of the polynucleotide is linked to a hairpin which prevents said end from entering the pore. In this case, the only single-stranded polynucleotide end which can be captured by the nanopore is the 3' end, resulting in 3' to 5' movement of said polynucleotide through the said pore, as when the enzyme is a replicative helicase.

A "nucleic-acid binding enzyme" according to the invention is any type of enzyme which is capable of binding a nucleic acid molecule and exerting its activity onto said molecule. Advantageously, the activity of the enzyme of the invention is sensitive to forces exerted on the polynucleotide. In a preferred embodiment, the enzyme is activated when a force is exerted on the said polynucleotide. This activation can be either direct or indirect. The activation is "direct" when the force exerted by the pore is required by the enzyme to overcome a barrier to its activity. For example, the T4 and the T7 holoenzymes cannot perform elongation in strand displacement mode because the DNA fork upstream of the holoenzyme generates a regression pressure which inhibits the polymerization-driven forward motion of the holoenzyme (Manosas et al., Nucl Acids Res, 40(13):6174-6186, 2012). Likewise, the T4 helicase gp41 is a passive helicase which cannot unwind DNA alone (Lionnet et al., Proc Natl Acad Sci USA, 104(50): 19790-19795, 2007). Thus, the T4 and T7 holoenzymes, like gp41, need direct assistance from the pore to unwind DNA. On the other hand, the activation is "indirect" when the action of the pore is merely required for presenting the enzyme with the correct substrate. For example, a polynucleotide having a covalent crosslink between the two strands at one extremity may be denatured by the action of the nanopore until the crosslink stops the unwinding process. The resulting molecule is a partially open fork, which is the preferred substrate for rewinding helicases such as UvsW and RecG (Manosas et al., Nat Commun, 4: 2368, doi: 10.1038/ncomms3368, 2013).

In one embodiment, the nucleic-acid binding enzyme of the invention is a molecular motor which is capable of causing the fork to move. When a molecular motor is present, the molecular motor can bind to the polynucleotide and cause the fork to move. This has the effect of causing the polynucleotide to move through the nanopore, thus resulting in variations of the current specific for the nucleotides present in the nanopore.

The molecular motor can be any suitable molecule which is capable of causing movement of the polynucleotide. Movement of the polynucleotide can be through contraction, stretching, rotation and/or supercoiling. This, in turn, causes movement of the polynucleotide through the pore. Suitable molecular motors include topoisomerases, polymerases (such as DNA polymerase), helicases, recombinases, chromatin and chromatin-remodeling factors, regulation factors, translocases and restriction-modification enzymes. For example, a DNA translocase such as Fts could be used or a type I restriction-modification enzyme such as EcoR.1241. Other suitable enzymes are Rad54, Topoisomerase 1, UvrD, RecQ and gp41. As further examples of suitable enzymes, one may cite e.g. helicases, including a UVrD helicase, a recBCD helicase, E. coli UvrD helicase, Tte-UvrD helicase, T7 gp4 helicase, RecBCD helicase, DnaB helicase, MCM helicase, RTEL1 helicase, Rep helicase, RecQ helicase, PcrA helicase, T4 UvsW helicase, SV40 large T antigen helicase, Herpes virus helicase, yeast Sgs1 helicase, DEAH_ATP-dependent helicases and Papillomavirus helicase E1 protein and homologs thereof. Preferably, the molecular motor is a DNA manipulating enzyme.

In a first aspect, the said molecular motor is a replicative helicase or a replicative polymerase. As used herein, a "replicative helicase" is an enzyme that unwinds DNA during chromosomal replication. It uses energy from nucleoside triphosphate hydrolysis to translocate along one strand of the duplex DNA and displace the complementary strand. Replicative helicases according to the invention include such enzymes as DnaB, the Mcm4,6,7 heterotrimer, gp41, RTEL1, RecQ, gp4 and the like. A "replicative polymerase" as used herein is an enzyme which exhibits rapid and processive primer extension DNA synthesis, but requires external help to unwind the duplex nucleic acid. Examples of replicative polymerases comprise the T4 DNA polymerase, the T7 DNA polymerase, DNA polymerase α, E. coli Pol II, E. coli Pol II, RB69 Polymerase gp43, and similar enzymes. Preferred enzymes according to this embodiment include such enzymes as the T4 DNA polymerase, the T7 DNA polymerase and the T4 helicase gp41 and the like.

It is known that these enzymes are unable to perform strand displacement without assistance. On the other hand, the inventors have shown that the strand displacement activity is strongly stimulated by an applied force (Lionnet et al., Proc Natl Acad Sci USA, 104(50): 19790-19795, 2007; Manosas et al., Nucl Acids Res, 40(13):6174-6186, 2012).

Such a force is provided in this embodiment by the voltage applied to the nanopore. According to this embodiment, when the enzyme is loaded onto the polynucleotide of the invention, the said enzyme is unable to unwind the said polynucleotide on its own, even in the presence of nucleotides triphosphate. However, the capture of a strand of the polynucleotide by the pore provides the force necessary for the activation of the enzyme. For example, under these conditions, a replicative polymerase will start synthesizing a new strand while displacing the fork, thus leading to the unwinding of the nucleic acid molecule in front of the fork. A replicative helicase will only start unwinding at a sustained rate when a strand of the polynucleotide enters the nanopore. The voltage applied on the nanopore exerts a force on the single strand of the polynucleotide thus trying to pull it apart from the double-stranded part of the said polynucleotide. In this embodiment, the activation of the enzyme is coordinated with the passage of the strand into the nanopore. In turn, the activity of the enzyme limits the rate of translocation of the said strand into the nanopore, thus generating a nucleotide-dependent signal which can be read and identified.

According to this embodiment, the voltage used is typically from + 100 mV to + 400 mV. The voltage used is preferably in range comprised between + 120 mV and + 240 mV. More preferably, the said voltage is between + 160 mV and + 240 mV. Most preferably, said voltage is comprised between + 180 mV and + 240 mV.

The skilled person will immediately realize that the polynucleotide and the enzyme may be mixed with the nanopore without any risk of starting unwinding. It is only when the voltage is applied that the polynucleotide is pulled into the pore and that the enzyme is activated. In this embodiment, the molecular motor of the invention is thus directly activated by the pore.

Since a substantial voltage drive is required according to this embodiment for activating the enzyme, it will be easily understood that a decrease of the said voltage will result in the inactivation of the said enzyme. Thus, in a further preferred embodiment, the voltage applied is decreased under a minimal value, e.g. 10 mV, after the polynucleotide has been substantially unwound. This results in the inactivation of the enzyme and the reformation of the double-stranded nucleic acid.

In this regard, it may be particularly useful to use a replicative polymerase such as the T4 DNA polymerase or the T7 DNA polymerase, since such an enzyme will not only cease synthesizing a new strand when voltage is reduced, but will actually start removing the strand just assembled. This will ensure efficient renaturation of the denatured polynucleotide, thus obtaining the substrate for the next round of unwinding of the molecule. Indeed, the inventors have found that the T4 DNA polymerase and the T7 DNA polymerase, which are not known to have any strand displacement activity in conditions where no tension is applied to the double-stranded template, are capable of removing the downstream nucleic acid during polymerisation when the double-stranded hairpin is under a force ≥ 10 pN (Manosas et al., Nucl Acids Res, 40(13):6174-6186, 2012). "T4 DNA Polymerase" and "T7 DNA Polymerase" herein refer to both the monomeric enzyme and the holoenzyme.

Since the driving voltage acts as a force, the polymerase is activated by a substantial voltage drive. When the voltage is reduced, the activity of the polymerase is thus switched towards the exonuclease mode, thus degrading processively the newly synthetized strand as it travel backwards. This motion is accompanied by the concomitant movement of the fork, thus promoting the renaturation of the denatured polynucleotide and the pulling back of the strand engaged into the nanopore. Replicative polymerases such as the T4 DNA polymerase and T7 DNA polymerase are thus particularly suited for carrying out the method of the invention.

According to this particular embodiment, it is possible to perform multiple cycles of pairing and unpairing and thus to improve the signal/noise ratio by simply controlling the voltage.

For example, the nanopore-driving voltage can be periodically modulated, with the voltage being turned high for a defined period of time and then switched back to a low value for a longer period. The on time of the high voltage defines the maximum extent of the unwinding of the molecule. Advantageously, this extent is shorter than the molecule, thus allowing the refolding of the molecule once the voltage is lowered. The pairing of the two strands will stop when the two DNA arms cease to be complementary, i.e. at the starting point of the molecule with the 5' arm still engaged in the pore. Upon the voltage increase of the next cycle, the enzyme will unwind the polynucleotide and restart a new cycle. This process will thus provide multiple translocation of the DNA strand in the nanopore.

In order to ensure that the enzyme does not completely unwind the polynucleotide, but rather stays on the molecule, modified nucleotides may be added at one extremity of the said polynucleotide. Preferably, the said modified nucleotides are added at the extremity of the polynucleotide which is not captured by the nanopore. Under unwinding conditions, the enzyme will be stalled on these nucleotides until the voltage is reduced. The fork will then move backward until it reaches the other end of the molecule, indicating that the system is ready for another round of high and low voltage.

The advantages of the said process are numerous. Most remarkable is its great simplicity. Indeed, the voltage can be increased or decreased independently of the molecule capture, while the enzyme will synchronize directly with the voltage modulation.

In another aspect, the said molecular motor is a rewinding helicase. By "rewinding helicase", it is herein referred to a helicase with strand annealing activity. A rewinding helicase is thus a helicase which is capable of rewinding, or annealing, complementary strands of nucleic acids in the presence or absence of nucleotide triphosphate. Rewinding helicases are well known in the art (Wu, J Nucleic Acids, 2012: 140601, 2012, doi: 10.1155/2012/140601) and encompass such enzymes as human HARP, human AH2, T4 UvsW, E. coli RecG, human WRN, Mycobacterium tuberculosis XPB, Saccharomyces cerevisiae Ded1, human RECQ4, human BLM, S. cerevisiae Sgs1, among others.

These enzymes are inefficient to unwind a double-stranded polynucleotide but are extremely efficient to re-anneal partially open double-stranded polynucleotide. These enzymes load onto an open fork and are capable of re-annealing the polynucleotide. However, once the polynucleotide is completely re-annealed the enzyme falls off (Manosas et al., Science, 338(6111): 1217-1220, 2012; Manosas et al., Nat Commun,4: 2368, 2013).

In this aspect of the invention, the presence of the enzyme's substrate is required for, and is sufficient, with the optional addition of nucleotide triphosphate, for activating the strand annealing activity of the rewinding helicase. The activation of the enzyme is thus indirectly caused by the pore.

In a preferred embodiment of the invention, the enzyme is activated by being provided with a partially unzipped polynucleotide. Preferably, said polynucleotide is unzipped by the action of the nanopore. The capture of a strand by the nanopore results in the rapid translocation of the said strand into the nanopore, while the other strand remains in the original compartment. Thus, more preferably, the capture of a strand of the said polynucleotide leads to the unwinding of the polynucleotide and the activation of the enzyme.

In order to ensure that the polynucleotide is not totally unwound by the action of the pore, but is stalled in a partially open fork configuration, it may be advantageous to add a crosslink between the two strands at one extremity of the molecule. Preferably, the crosslink is introduced at the extremity which is not pulled into the nanopore. In that case, a strand is threaded into the nanopore without any element to slow it down, thus unwinding the polynucleotide. However, this unwinding stops at the position of the crosslink which is a covalent bonding. Once the molecule is blocked in the nanopore, it contains a stalled fork, which corresponds to the preferred substrate for a rewinding helicase. The rewinding helicase of the invention will then load onto the substrate and re-anneal the said polynucleotide of the invention until the whole polynucleotide is annealed. The rewinding of the polynucleotide will be accompanied by traction of the strand out of the pore, thus generating a signal dependent upon the nature of the nucleotide traversing the pore.

The rewinding helicase of the invention does not bind to a nucleic acid molecule which is completely annealed. Therefore, in a preferred embodiment, once the whole polynucleotide has been annealed by the rewinding polymerase, the said enzyme falls off the duplex polynucleotide. This behavior of the enzyme provides a way to repeat translocation of the same sequence through the pore. In a further preferred embodiment, the cycle of unwinding/rewinding is repeated several times. The dissociation of the enzyme with the polynucleotide allows a new cycle of denaturation/renaturation to start: a strand of the polynucleotide is grabbed by the pore, which pulls on the said strand until the unwinding of the molecule reaches the crosslink; at this point, the rewinding helicase loads onto the substrate and reanneals the polynucleotide.

Therefore this embodiment of the method of the invention affords an easy and efficient way to perform multiple rounds of unwinding and rewinding and thus improve the signal/noise ratio.

Rewinding helicases are capable of rewinding a polynucleotide against an opposing force. For example, the inventors have shown that UsvW and RecG helicases both display enough strength to extrude a polynucleotide out of a nanopore (Manosas et al., Science, 338(6111): 1217-1220, 2012; Manosas et al., Nat Commun, 4: 2368, 2013). This situation is particularly advantageous, because there is no need to decrease the voltage. Therefore, the entire process can be performed at the same voltage. The method is typically carried out with a voltage applied across the membrane and pore. The voltage used is typically from -400mV to +400mV. The voltage used is preferably in a range having a lower limit selected from -400 mV, - 300mV, -200 mV, -150 mV, -100 mV, -50 mV, -20mV and 0 mV and an upper limit independently selected from +10 mV, + 20 mV, +50 mV, +100 mV, +1 0 mV, +200 mV, +300 mV and +400 mV. The voltage used is comprised preferably between 50 mV and 300 mV, more preferably between 100 mV and 250 mV, and most preferably between 150mV and 250mV.

However, it may also be advantageous in certain embodiments to decrease the voltage in order to enable the rewinding helicase to load more efficiently onto the substrate polynucleotide.

A "nanopore" according to the invention is an aperture, gap, or other hole in a support structure.

Preferably, the nanopore is an aperture in a membrane allowing the transport of ions from one side of the membrane to the other. The nanopore may be described as having longitudinal and transverse dimensions. The longitudinal dimensions of the nanopore determine the distance that a polynucleotide must travel to pass through the pore, i.e., the thickness of the pore. The transverse dimensions of the nanopore determine the largest species that can enter the pore, i.e., the width of the pore: the pore must be wide enough to accommodate the polynucleotide which is to sequenced. Desirably, the longitudinal dimension of the nanopore is small enough to restrict the polymer to a discrete set of measurable conformations. The longitudinal dimension is also desirably smaller than the conformation being observed. Nanopores useful in the invention typically range in transverse dimension from 1-1000 nm, e.g., at most 750, 500, 400, 300, 250, 200, 150, 100, 90, 80, 70, 60 50, 40, 30, 20, 10, 9, 8, 7, 6, 5, 4, 3, or 2 nm and at least 2, 3, 4, 5, 6, 7, 8, 9, 10, 20, 30, 40, 50, 60, 70, 80, 90, 100, 150, 200, 250, 300, 400, 500, or 750 nm. Nanopores useful in the invention typically range in longitudinal dimension from 1-1000 nm, e.g., at most 750, 500, 400, 300, 250, 200, 150, 100, 90, 80, 70, 60 50, 40, 30, 20, 10, 9, 8, 7, 6, 5, 4, 3, or 2 nm and at least 2, 3, 4, 5, 6, 7, 8, 9, 10, 20, 30, 40, 50, 60, 70, 80, 90, 100, 150, 200, 250, 300, 400, 500, or 750 nm. The dimensions of the nanopore employed will depend on the type of polynucleotide being probed and the conditions of the polynucleotide solution.

Preferably, the limiting aperture of the nanopore is just sufficient for accommodating single stranded polynucleotide.

Nanopores can be made of synthetic materials (solid-state nanopores) or derived from a protein or a protein complex (protein nanopore).

In a first embodiment, the nanopore is a solid-state nanopore. "Solid-state" is used herein to refer to materials that are not of biological origin. By biological origin is meant derived from or isolated from a biological environment such as an organism or cell, or a synthetically manufactured version of a biologically available structure. Solid-state encompasses both organic and inorganic materials including, but not limited to, microelectronic materials, insulating materials such as Si₃N₄, Al₂O₃, and SiO, organic and inorganic polymers such as polyamide, plastics such as Teflon® or elastomers such as two-component addition-cure silicone rubber, and glasses, although there is no specific limitation to the materials that may be used according to the invention. More preferably, solid-state includes materials such as silicon nitride, silicon oxide, silicon carbide, mica, polyimide (PI), lipids, poly(ethyleneterephthalate) (PET), polycarbonate(PC), poly(vinylidenefluoride) (PVDF), and grapheme.

A "solid-state nanopore" according to the invention is thus a nanopore made of any appropriate material, such as, but not limited to, silicon nitride, silicon oxide, mica, polyimide (PI), lipids, poly(ethyleneterephthalate) (PET), polycarbonate (PC), poly(vinylidenefluoride) (PVDF), and graphene. Solid-state nanopores are particularly advantageous because they can be dimensioned so that only a single stranded polynucleotide can pass through the nanopore aperture at a given time. Solid-state nanopores are well-known in the art (Wanunu and Meller, Nano Lett, 7(6):1580-1585, 2007; WO 00/78668; WO 2009/020682) There is no need to detail them further herein.

In another embodiment, the nanopore of the invention is a protein pore. Preferably, the nanopore of the invention is a transmembrane protein pore. A transmembrane protein pore is a polypeptide or a collection of polypeptides that permits hydrated ions to flow from one side of a membrane to the other side of the membrane. In the present invention, the transmembrane protein pore is capable of forming a pore that permits hydrated ions driven by an applied potential to flow from one side of the membrane to the other. The transmembrane protein pore preferably permits analyte such as nucleotides to flow from one side of the membrane, such as a lipid bilayer, to the other. The transmembrane protein pore allows a polynucleotide, such as DNA or RNA, to be moved through the pore.

Transmembrane protein pore offer several advantages for single-molecule polynucleotide analysis. First, cells can produce large numbers of biological nanopores with an atomic level of precision that cannot yet be replicated by the semiconductor industry; second, X-ray crystallography has provided information about the nanopore structure at ångström length scales; third, established genetic techniques (notably site-directed mutagenesis) can be used to tailor the physical and chemical properties of the nanopore; and fourth, remarkable heterogeneity is observed among biological nanopores in terms of size and composition.

The transmembrane protein pore may be a monomer or an oligomer. The pore is preferably made up of several repeating subunits, such as 6, 7 or 8 subunits. The pore is more preferably a heptameric or octameric pore.

The transmembrane protein pore typically comprises a barrel or channel through which the ions may flow. The subunits of the pore typically surround a central axis and contribute strands to a transmembrane β-barrel or channel or a transmembrane α-helix bundle or channel.

The barrel or channel of the transmembrane protein pore typically comprises amino acids that facilitate interaction with analyte, such as nucleotides, polynucleotides or nucleic acids. These amino acids are preferably located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids. Transmembrane protein pores for use in accordance with the invention can be derived from ß-barrel pores or α-helix bundle pores, ß-barrel pores comprise a barrel or channel that is formed from ß-strands. Suitable ß-barrel pores include, but are not limited to, ß-toxins, such as α-hemolysin, anthrax toxin and leukocidins, and outer membrane proteins/porins of bacteria, such as *Mycobacterium smegmatis* porin (Msp), for example MspA, outer membrane porin F (OmpF), outer membrane porin G (OmpG), outer membrane phospholipase A and Neisseria autotransporter lipoprotein (NalP). α-helix bundle pores comprise a barrel or channel that is formed from a-helices. Suitable α-helix bundle pores include, but are not limited to, inner membrane proteins and outer membrane proteins, such as WZA and ClyA toxin. Preferably, the transmembrane protein pore may be derived from Msp or from α-hemolysin (α-HL) (see, for example: Akeson et al., Biophys. J, 77: 3227-3233, 1999; Meller et al., Proc Nat Acad Sci, 97: 1079-1084, 2000; Braha et al. Nat. Biotech, 18(9): 1005-1007, 2000; Meller et al., Phys. Rev. Lett, 86: 3435-3438, 2001; Meller et al., Electrophoresis, 23: 2583-2591, 2002; Bates et al., Biophys. J, 84: 2366-2372, 2003; Zwolak et al., Rev Mod Phys, 80: 141-165, 2007). Even more preferably, the transmembrane protein pore should enable the discrimination among different DNA bases. In this respect, it is particularly advantageous to use a variant of the MpsA protein described in Butler et al. (Proc Natl Acad Sci USA, 105: 20647-20652, 2008).

When a nanopore is present in an electrically insulating membrane, it can be used as a single-molecule detector. Thus, in a preferred embodiment, the nanopore of the invention is coupled to a membrane.

A "membrane" according to the invention is a thin film which separates two compartments and prevents the free diffusion of ions and other molecules between the said compartments.

Any membrane may be used in accordance to the invention. Suitable membranes are well-known in the art and include amphiphilic layers and solid-state layers, among others. In a first embodiment, the said membrane is a solid-state membrane, i.e. a layer prepared from solid-state materials, in which one or more apertures is formed. The membrane may be a layer, such as a coating or film on a supporting substrate, or it may be a free-standing element. Alternatively, it may be a composite of various materials in a sandwich configuration. The thickness of the membrane may vary, and in particular, the membrane may be considerably thinner in the region containing the aperture. In embodiments, in which the membrane is a layer on a supporting substrate, the supporting substrate includes an appropriately positioned gap, so that the portion of the membrane containing the aperture spans the gap.

It will be immediately apparent to the person of skills in the art that solid-state membranes are advantageously coupled with solid-state nanopores. Indeed, solid-state nanopores are routinely produced in solid-state membranes such as the ones described above, by methods such as focused electron or ion beams (Dekker, Nature Nanotech, 2: 209-215, 2007; Kocer et al., Biosens Bioelectron, 38(1):1-10, 2012).

In another embodiment, the membrane of the invention is an amphiphilic layer. An amphiphilic layer is formed of amphiphilic molecules, i.e. molecules possessing both hydrophilic and lipophilic properties. Such amphiphilic molecules may be either naturally occurring, such as phospholipids, or synthetic. Examples of synthetic amphiphilic molecules include such molecules as poly(n-butyl methacrylate-phosphorylcholine), poly(ester amide)-phosphorylcholine, polylactide-phosphorylcholine, polyethylene glycol-poly(caprolactone)-di- or tri-blocks, polyethylene glycol-polylactide di- or tri-blocks and polyethylene glycol-poly(lactide-glycolide) di-or tri-blocks. Preferably, the amphiphilic layer is a lipid bilayer. Lipids bilayers are models of cell membranes and have been widely used for experimental purposes. For example, lipid bilayers have been used to study ion channels properties (Heimburg, Biophys Chem, 150(1-3): 2-22, 2010). Such lipid bilayers may be either artificial or of cellular origin.

Thus the method of the invention may be carried out using (i) an artificial membrane, such as a lipid bilayer, comprising a pore; (ii) an isolated, naturally-occurring lipid bilayer comprising a pore, or (iii) a cell having a pore inserted therein. The method is preferably carried out using an artificial bilayer, such as a lipid bilayer. Methods for forming lipid bilayers are well-known in the art (see e.g. Montal 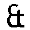 Mueller, Proc. Natl. Acad. Sci. USA, 69(12): 3561-3566, 1972; WO 01/070419; WO 2006/110350; WO 2006/100484; WO 2008/102121; WO 2009/077734; Phung et al., Biosens Bioelectron, 26(7):3127-3135, 2011; Hirano-Iwata et al., Anal Sci., 28(11): 1049-1057, 2012).

The sequencing method of the invention may be carried out using any apparatus that is suitable for investigating a membrane/pore system in which a pore is present in or inserted into a membrane. The method may be carried out using any apparatus that is suitable for nanopore sensing. For example, the apparatus comprises a chamber comprising an aqueous solution and a barrier that separates the chamber into two reservoirs. The barrier has an aperture in which the membrane containing the pore is formed. The polynucleotide may be present in either of the two reservoirs of the chamber.

The application of a constant DC voltage between the two reservoirs results in a baseline ionic current that is measured. If a polynucleotide is introduced into a reservoir, it may pass through the fluidic channel and change the observed current, due to a difference in conductivity between the electrolyte solution and polynucleotide. The magnitude of the change in current depends on the volume of electrolyte displaced by the polynucleotide while it is in the fluidic channel. The duration of the current change is related to the amount of time that the polynucleotide takes to pass through the nanopore constriction. In the case of DNA translocation through a nanopore, the physical translocation may be driven by the electrophoretic force generated by the applied DC voltage.

The method of the invention involves measuring the current passing through the pore as the target polynucleotide moves with respect to the pore. Therefore the apparatus also comprises an electrical circuit capable of applying a potential and measuring an electrical signal across the membrane and pore. The methods may be carried out using a patch clamp or a voltage clamp. The method preferably involves the use of a voltage clamp.

Nanopore sensing relies on ions going through the nanopore. When the nanopore is formed in an insulating membrane separating two chambers filled with conductive electrolyte, charged molecules such as ions can be driven through the pore under an applied electric potential (a process known as electrophoresis), thereby modulating the ionic current through the nanopore. During the interaction of the polynucleotide and the pore, the polynucleotide affects the current flowing through the pore in a manner specific for that polynucleotide. This current reveals useful information about the structure and dynamic motion of the molecule.

Thus in a preferred embodiment, a polynucleotide detection apparatus may include the nanopore described above, said nanopore being optionally coupled to a membrane; a reservoir for containing the sample that translocated through the nanopore; and a power supply to form an electric field around the nanopore in order to move the target polynucleotide in the sample. In other words, the nanopore of the invention, optionally coupled to a membrane, is part of a detector specific for polynucleotides. The said detector is a structure that provides a readable signal in response to the presence, the absence or the characteristics of the polynucleotide.

In preferred embodiments, the detector detects the polynucleotide using electrical means. In this respect, the method of the invention comprises advantageously providing at least one nanopore in a membrane that is disposed adjacent or in proximity to an electrode. The electrode can be adapted to detect a current passing through the nanopore. The method can further include inserting a polynucleotide or portion thereof into the nanopore and varying a voltage applied across the nanopore and/or across the membrane. In some cases, the method includes measuring the current at a plurality of voltages to detect the presence, the absence or the characteristics of the polynucleotide. In some embodiments, the current at a plurality of voltages comprises an electronic signature and further comprises comparing the electronic signature to a plurality of reference electronic signatures to identify the characteristics of the polynucleotide.

The nanopore may be formed or otherwise embedded in a membrane disposed adjacent to a sensing electrode of a sensing circuit, such as an integrated circuit. The integrated circuit may be an application specific integrated circuit (ASIC). In some examples, the integrated circuit is a field effect transistor or a complementary metal-oxide semiconductor (CMOS). The sensing circuit may be situated in a chip or other device having the nanopore, or off of the chip or device, such as in an off-chip configuration. The semiconductor can be any semiconductor, including, without limitation, Group IV (e.g., silicon) and Group III-V semiconductors (e.g., gallium arsenide). A variety of electronic devices are available which are sensitive enough to perform the measurements used in the invention, and computer acquisition rates and storage capabilities are adequate for the rapid pace of sequence data accumulation.

The method of the invention is typically carried out in the presence of a buffer. In the apparatus discussed above, the buffer is present in the aqueous solution in the chamber. Any buffer may be used in the method of the invention. One suitable buffer is Tris-HCl buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The method of the invention is typically carried out at from 0°C to 100°C, from 15°C to 95°C, from 16°C to 90°C, from 17°C to 85°C, from 18°C to 80°C, 19°C to 70°C, or from 20°C to 60°C. The method may be carried out at room temperature. The method is preferably carried out at a temperature that supports enzyme function, such as about 37°C.

The practice of the invention employs, unless other otherwise indicated, conventional techniques or protein chemistry, molecular virology, microbiology, recombinant DNA technology, and pharmacology, which are within the skill of the art. Such techniques are explained fully in the literature. (See Ausubel *et al.,* Current Protocols in Molecular Biology, Eds., John Wiley 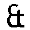 Sons, Inc. New York, 1995; Remington's Pharmaceutical Sciences, 17th ed., Mack Publishing Co., Easton, Pa., 1985; and Sambrook et al., Molecular cloning: A laboratory manual 2nd edition, Cold Spring Harbor Laboratory Press - Cold Spring Harbor, NY, USA, 1989).

The examples below will enable other features and advantages of the present invention to be brought out.

### Legends of the Figures

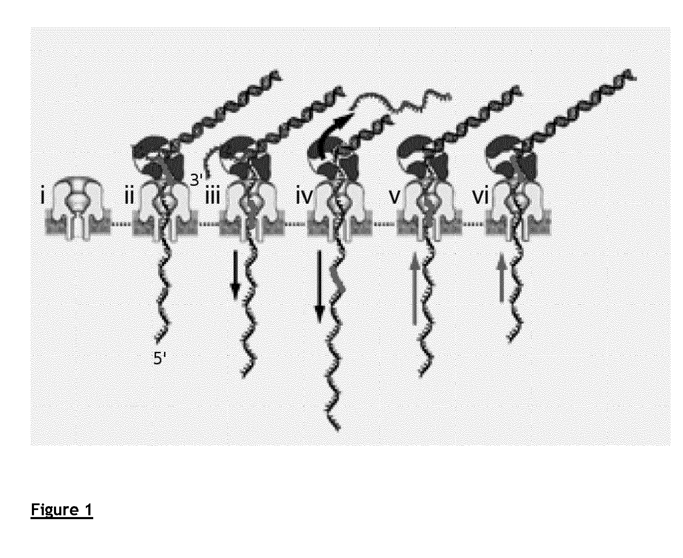

**Fig. 1****:** Principle of the synchronization of the DNA strand capture by the nanopore and the start of the polymerase as described by Cherf et al. (Nat. Biotechnol., 30(4): 344-348, 2012). (i) Nanopore waiting to capture a molecule. (ii) A molecule template is threaded through the nanopore by its 5' tail with a bound polymerase, a protecting oligonucleotide (in orange) and a primer hybridized after the protecting oligonucleotide. The protecting oligonucleotide has a flap which is not hybridized to the template. (iii) A strong forward voltage is applied unzipping the protecting oligonucleotide and driving the polymerase in place with its primer. As this occurs, the template advances in the nanopore as can be detected by the passage of the template abasic sites (in blue) in the nanopore sensing area. (v) As the polymerase starts elongating its primer, the template is now extracted from the nanopore with the polymerase rate. (vi) Finally the elongation is stopped when the abasic bases reach the polymerase.

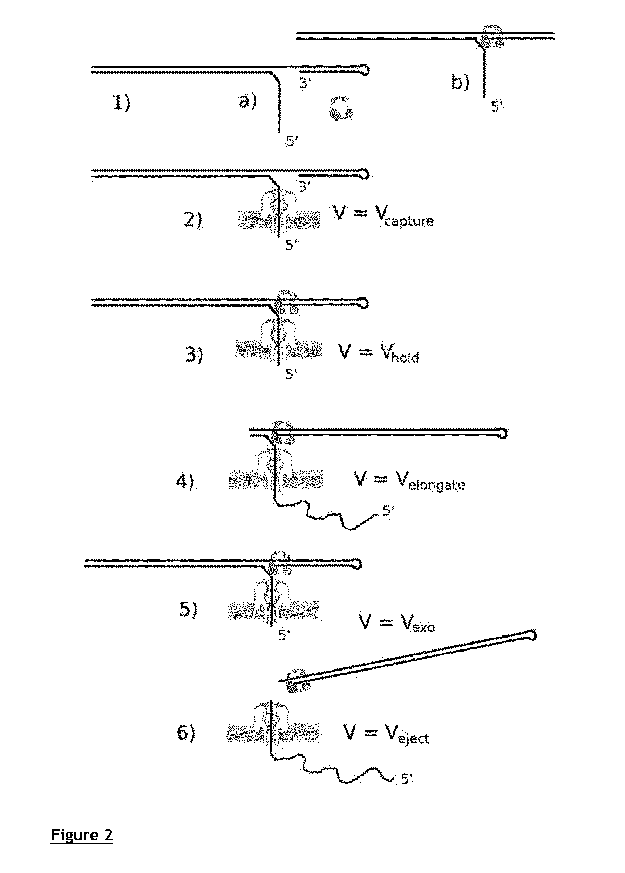

**Figure 2****:** Coupling replicative polymerase with nanopore. 1) DNA molecules prepared with a fork adaptors are placed in solution with replicative polymerase and dNTPs above the nanopore. The polymerase may or may not load on the primer. If it loads, the elongation is soon stalled by the lagging strand which needs to be displaced. Thus the polymerase cannot elongate this substrate in solution. 2) The 5' arm of the fork is capture by the nanopore with a driving voltage V_{capture} substantial. As the capture is detected, the voltage is lowered to V_{hold} a medium value exerting a moderate force on the strand not enough to unzip the molecule. 3) At some point the polymerase loads on the primer, since the nanopore helps pealing DNA, the polymerase is switched in its elongation mode and the lagging strand is extrude through the nanopore. 4) After some time or after the polymerase has been stopped by abasic sites, the driving voltage is very much reduced switching the polymerase in its exonuclease mode. The polymerase runs backward pulling back the lagging strand in the naopore until the polymerase reaches the fork origin where the two strands are not anymore complementary. Phase 3) and 4) can be iterated several times in cycles until the molecule can be expelled by applying a high driving voltage V_{eject}.

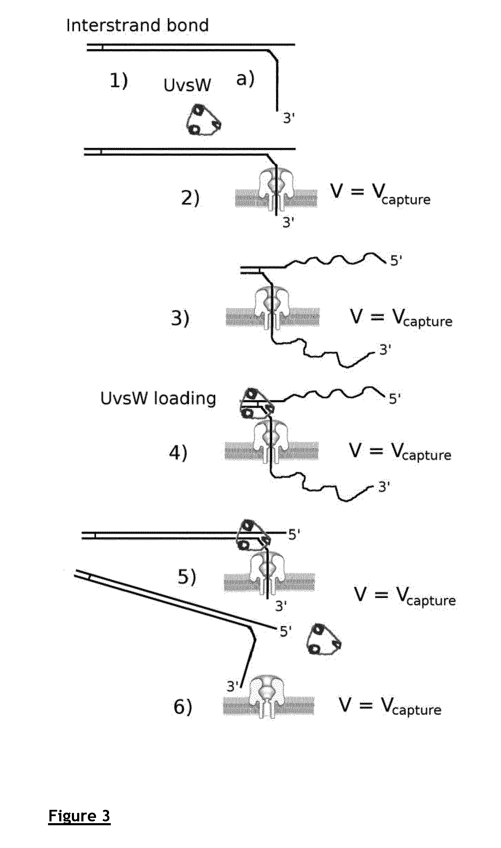

**Figure 3****:** Coupling rewinding helicases, e.g. UvsW or RecG, with nanopore. A dsDNA substrate having a fork at one end provides the staring molecule 1) which is ligated to a piece of dsDNA having a crosslink between the two strands. This adaptor may be a hairpin to prevent its entry in the nanopore. Both UvsW and RegG have no effect on this substrate. Once one arm is captured by the nanopore 2), the driving voltage is set to a value strong enough to completely unwind the dsDNA molecule until the nanopore bumps in the crosslink holding the two strands together 3). At the stage the molecule is stalled in the nanopore until a UvsW (or a recG) helicase loads on the dsDNA substrate 4). When this occurs, the enzyme starts re-annealing the dsDNA extruding the ssDNA from the nanopore. It does that in a very processive manner until it reaches the fork origin 5). There depending on the arm length the helicase might either detach or completely stiff off the DNA from the nanopore.

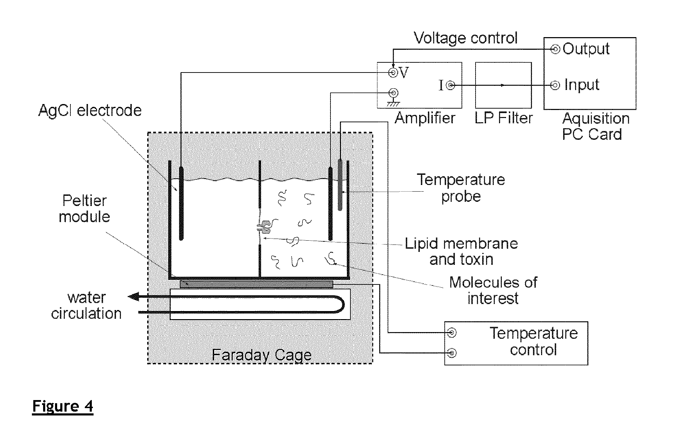

**Figure 4****:** Setup used in the experimental example

### Experimental example

The cell: The cell is composed of two compartments (called cis and trans compartments) connected by a U-shaped tube. This tube is terminated at one end (cis side) by a hole of 10-30-micron diameter on which the lipid bilayer will be formed. This homemade cell is made of Teflon. The chambers and the tubes are filled with the buffer solution: usually 1 M KCl and 10 mM Tris-HCl at pH 8.5 or 5 mM HEPES at pH 7.5. But for the good processing of the enzymes it is adapted using 300 to 600 mM KOAc, 10 mM Mg(OAc)₂ and 25mM TrisOAc at pH 7.5. The AgCl electrodes are plunged either directly in the KCl buffer or in a 1 M KCl solution itself connected to the acetate buffer using agar-bridges. One of the electrodes (cis side) is connected to the ground. The other (trans side) is connected to the potential applied.

When the cell is filled with buffer, after checking the electrical connection between the two chambers, the lipid bilayer is painted on the 10-30-micron hole. The lipids used are most commonly diphytanoyl phosphatidylcholine (from Avanti Lipids). The bilayer is checked measuring its capacitance (about 10 pF for 10-micron hole) and its resistance (1 GOhm to get a giga seal). Then the toxin monomers (α-Hemolysin for instance) are added to the cis side at a final concentration of about 10 pg/ml. The toxin insertion is observed by applying a voltage of +100 mV generating an ionic current of 100 pA (at room temperature) when a single protein pore is inserted in the bilayer. As soon as a toxin is inserted, the cis chamber is rinsed by circulation of buffer (10 times the volume of the chamber) to avoid any further insertion and Current-Voltage characteristic of the inserted nanopore is measured. The toxin should have a rectification of about 20 % of its current meaning that the current with backward bias is 20 % smaller than with forward bias. It should as well be stable at any voltage used in the experiment. If the toxin is not satisfying the lipid membrane is reformed and the toxin is added again in cis chamber. When a usable toxin is inserted, the system is heated or cooled is set to desired temperature using a peltier module imbedded in a copper block holding the Teflon cell described above. The Peltier module is controlled via a Newport (model 3040) temperature controller.

The ionic current is measured and the voltage bias is applied using a patch clamp amplifier (typically Axopatch 200B, Axon Instrument, Molecular devices). The ionic current amplified and converted, is then filtered using a 100 kHz low pass filter (Khron Hite 3361) prior digitization (1 Msamples/s) and recording using a DAQ card and homemade programs written in LabView.

The molecules or molecules assemblies are added to the cis side of the cell. Applying a positive voltage will drag the DNA molecules toward the trans side. Every time a molecule is inserted in the nanopore, it blocks the ionic current. This sudden rise of the pore resistance is then used as a trigger to fire the applied force sequence. Starting from this point in time it is possible to apply a force too low to start the enzyme processivity but holding the DNA molecule in the pore , or high enough to start the enzyme processivity which will move the DNA in the pore at a constant speed and thus read the current to deduce the bases sequence.

## Claims

1. A method of sequencing a polynucleotide, comprising:
a) contacting said polynucleotide with a nanopore and a nucleic-acid binding enzyme;
b) introducing said polynucleotide into the said pore, resulting in the activation of the activity of the said enzyme;
c) allowing said polynucleotide to move with respect to the said pore, wherein the movement of said polynucleotide is controlled by the activity of the said enzyme;
d) monitoring the signal associated with the movement of the said polynucleotide with respect to the said pore, thereby generating the sequence of the said polynucleotide;
e) switching off the activity of the said enzyme; and
f) repeating steps a) to e).

2. The method of claim 1, wherein the said polynucleotide is a double-stranded nucleic acid.

3. The method of claim 1 or 2, wherein one end of said polynucleotide is attached to a hairpin.

4. The method of any one of claims 1 to 3, wherein said polynucleotide contains abasic sites or LNA bases.

5. The method of any one of claims 1 to 4, wherein said polynucleotide moves through the said pore.

6. The method of any one of claims 1 to 5, wherein step b) further comprises applying a voltage to the said pore.

7. The method of any one of claims 1 to 6, wherein the said enzyme is a molecular motor.

8. The method of claim 7, wherein the said molecular motor is a replicative helicase, notably grp41, or a replicative polymerase, notably T4 DNA polymerase or T7 DNA polymerase.

9. The method of claim 8, wherein the movement of the said polynucleotide in step c) results in the unwinding of the said polynucleotide.

10. The method of claim 9, wherein the switching off of the activity of the enzyme results in the rewinding of the polynucleotide.

11. The method of claim 8, wherein the said molecular motor is a rewinding helicase, notably UvsW or RecG.

12. The method of claim 11, wherein the movement of the said polynucleotide in step c) results in the unwinding of the said polynucleotide.

13. The method of claim 12, wherein the switching off of the activity of the enzyme results in the rewinding of the polynucleotide.

14. The method of any one of claims 11 to 13, wherein the polynucleotide comprises a crosslink between the two strands.

15. The method of any one of claims 1 to 14, wherein the said pore is a protein pore or a solid state pore.

16. The method of claim 15, wherein the protein pore is derived from Msp or
α -hemolysin (α-HL).

17. The method of any one of claims 1 to 16, wherein the said pore is coupled to a membrane.

18. The method of claim 17, wherein the said membrane is an amphiphilic layer or a solid state layer.

19. The method of claim 18, wherein the said membrane is lipid bilayer.

20. The method of any one of claims 1 to 19, wherein the said signal is an electric current passing through the pore.
